# EUROPEAN PATENT APPLICATION

(11) **EP 2 168 987 A1**
(43) Date of publication of application: **31.03.2010**
(21) Application number: 08164785.1
(22) Date of filing: 22.09.2008
(51) Int. Cl.: C07K 19/00, C07K 14/11, C07K 16/10, A61P 31/16

(54) **Multifunctional linker protein containing an antibody against hemagglutinin, a conserved influenza antigen and an immunostimulating carrier binding domain**

(71) Applicant: Mucosis B.V., 9747 AG Groningen (NL)
(72) Inventor: Leenhouts, Cornelis Johannes, 9753 KX Haren (NL); van Roosmalen, Maarten Leonardus, 9745 DG Groningen (NL); Schouten, Govert Johan, 2353 EM Leiderdorp (NL)
(74) Representative: Hatzmann, Martin

(57) **Abstract**

The invention relates to the field of immunology and vaccines. In particular, it relates to proteinaceous substances and the uses thereof in vaccines against infections caused by respiratory pathogens.

## Description

The invention relates to the field of immunology and vaccines. In particular, it relates to proteinaceous substances and the uses thereof in vaccines against infections caused by respiratory pathogens.

Influenza virus is one of the most important respiratory pathogens worldwide. Influenza virus typically infects 10-20% of the total worldwide population during seasonal epidemics, resulting in three to five million cases of severe illness and 250,000 to 500,000 deaths per year. In the US, influenza kills an average of 20,000 people per year, while an average of 114,000 influenza-related hospitalizations result in an estimated economic cost of $12 billion per year. Moreover, novel influenza strains appear occasionally in the human population, causing pandemics. The death toll of the 1917-1920 Spanish Flu pandemic resulted in over 50 million deaths worldwide, and the danger of a new influenza pandemic is always present. Vaccination is the best way to control influenza. However, in contrast to vaccination against many other pathogens, where the vaccine is given only one to three times in a lifetime, influenza vaccines have to be administered yearly.

Influenza displays an extraordinary capacity to change the antigenic characteristics of its two major membrane proteins, hemagglutinin (HA) and neuraminidase (NA). This occurs by the continuous selection away from the adaptive immune response established in the human population. Due to the high mutation rate of the virus, a particular vaccine formulation usually works for only about a year. The World Health Organization coordinates the contents of the vaccine each year to contain the most likely strains of the virus to attack the next year. Nowadays, conventional vaccines are vaccines consisting of three inactivated influenza viruses (two A-strains and one B). This trivalent influenza vaccine is re-formulated annually, based on influenza strains projected by the WHO to be prevalent in the upcoming flu season. For example, the annually updated trivalent flu vaccine for the 2007-2008 season consists of hemagglutinin (HA) surface glycoprotein components from influenza H3N2, H1N1, and B influenza viruses.

Influenza vaccines are dosed on the basis of their HA content, the major glycoprotein of the virus. Over 250 million doses of influenza vaccine are produced each year, with protective efficacy in children and elderly ranging between 60% and 80%. Hence, there is an unmet need for improved influenza vaccines especially in the vulnerable target groups such as children and elderly. Current approaches to improve influenza vaccines include:
a. the incorporation of an adjuvant
b. the incorporation of an additional influenza antigen component
c. strategies to elicit a local immune response at the site of the virus entry in the body, thus providing an extra line of defence by the vaccine.
Influenza vaccines are nowadays derived from virus grown in embryonated chicken eggs or tissue culture. Most influenza vaccines contain attenuated live virus, inactivated virus, split virus or HA subunit. New technologies are emerging that produce HA subunit material e.g. by using insect cells. A common way to increase the immunogenicity of vaccine components, especially of subunit vaccines, is to add an adjuvant. It is also commonly believed that adsorption or attachment either covalently or non-covalently of the vaccine components (antigens) to the adjuvant is advantageous. Aluminium salts (Alum) are the most commonly used adjuvants in human vaccines that are used for that purpose. However, influenza vaccines adsorbed onto Alum are toxic and therefore influenza vaccines approved for human use are primarily non-adjuvanted.

So far, there are no broad-spectrum vaccines available against influenza. In addition, vaccination coverage in the population is low in many countries despite the high morbidity and mortality that is associated with the seasonal outbreaks of influenza. The availability of an influenza vaccine that provides a broader spectrum of protection against influenza than the ones present in each yearly influenza vaccine, may be more attractive and thereby contribute to an increased effect and/or use of an influenza vaccine. In addition, a vaccine that is effective against multiple human influenza A strains regardless of their subtype may be considered for stockpiling as a precaution against new influenza pandemics.

It is therefore an aim of the present invention to provide improved influenza vaccines. In particular, the present inventors aimed to provide an adjuvanted influenza vaccine suitable for use in humans, which provides cross-protection against non-vaccine influenza types. Preferably, the molecular design of the novel vaccine allows for a convenient and cost-effective annual re-formulation.

At least some of these goals were met by the development of a multifunctional polypeptide comprising an influenza antigen component that is less susceptible to mutations, the polypeptide furthermore capable of being "loaded" with a variety of HA subtypes and being immobilized or attached onto an immunostimulating carrier.

Accordingly, the invention provides a proteinaceous substance comprising (i) at least one hemagglutinin (HA) binding domain (HBD) capable of non-covalently binding to multiple antigenic subtypes of HA, (ii) a conserved influenza protein, or an antigenic fragment thereof, and (iii) an immunostimulating carrier binding domain (IBD). Preferably, an antigenic fragment is capable of being presented by the major histocompatability complex (MHC) class I or II. Accordingly, the fragment will typically have a length of at least about 6 or 7 amino acid residues. Also provided is a nucleic acid sequence encoding the proteinaceous substance, and a host cell comprising the nucleic acid sequence. Further embodiments relate to a proteinaceous substance attached (via its IBD) to an immunostimulating carrier, and to an antigen-loaded immunostimulating carrier comprising multiple substances (e.g. antibody fragments) loaded with HA subtypes. The proteinaceous substance as disclosed herein allows for readily loading an immunostimulating carrier having adjuvant activity with different HA subtypes without modification of the HA antigen production process, while at the same time providing a broader (cross-)protection against other strains. The proteinaceous substance is also referred to as 'multifunctional protein anchor (abbreviated to 'Protan') linker'.

The relative position of the different domains within a proteinaceous substance of the invention can vary. The domains can be fused directly to each other or they may be spaced by a spacer sequence. Typically, the conserved influenza (membrane) protein or antigenic fragment thereof (or multiple copies thereof; see below) will be flanked on one side with the HBD and on the other side by the IBD, such that the HBD and IBD have easy access to their binding partners (HA antigen and immunostimulating carrier, respectively). In one embodiment, the at least one HBD and the IBD are located at, respectively, the C- and N-terminus of said proteinaceous substance. In another embodiment the at least one HBD and the IBD are located at, respectively, the N- and C-terminus the proteinaceous substance. The domains do not have to reside at the extreme ends of the proteinaceous substance; a stretch of one or more amino acid residues can be present at either end of the substance which are neither part of the HBD, nor of the IBD.
In a preferred embodiment, the HA-binding domain of a proteinaceous substance is capable of binding to multiple HA subtypes originating from influenza virus type A and/or type B. Exemplary HA subtypes include HA1, HA2, HA3, HA4, HA5, HA6, HA7, HA8, HA9, HA10, HA11, HA12, HA13, HA14, HA15 and HA16. In one embodiment, the HA-binding domain can recognize subtypes that are specific for seasonal influenza, e.g. HA1 and HA3. In another embodiment, it recognizes multiple subtypes specific for pandemic influenza, such as HA5, HA7, and HA9.
Suitable binding domains for influenza HA subtypes can be obtained in various ways:
i. selection of antibodies by immunization of mice, selection of monoclonals that show broad spectrum of binding to HA subtypes, determination of variable domain amino acid sequence;
ii. use of antibody phage display libraries to select scFv or nanobodies that show binding to multiple HA subtypes;
iii. antibodies described in the literature with known binding spectrum and known amino acid sequence of the variable domains.
Accordingly, in one aspect the invention provides a proteinaceous substance comprising (i) an antibody or functional fragment thereof capable of binding to multiple antigenic subtypes of HA, (ii) a conserved influenza protein, or an antigenic fragment thereof, and (iii) an immunostimulating carrier binding domain (IBD).
The HA binding domain is for example a single chain antibody (scFv) consisting of a variable light domain (VL) and a variable heavy domain (VH) connected by a spacer. Any type of spacer sequence may be used, provided that it does not contain potential proteolytic cleavage sites and that it allows sufficient flexibility to properly position the VL and VH domains in order to obtain a functional scFv. A suitable spacer to link VH and VL is spacer 218 (Whitlow, M et al. 1993. Protein Eng. 6: 989-995).

Watabe, T et al. (2007; Mol. Biol. Evol. 24: 1627-1638) describes four mouse monoclonal antibodies with known 3D structure that bind to HA. Two of the antibodies bind to slightly different epitopes on the receptor-binding region of HA (monoclonals HC19 and HC63, structures 2VIR.PDB and 1KEN.PDB) and two antibodies that bind on one epitope on a more membrane-proximal region on the side of HA that is not involved in receptor binding (HC45 and BH151, structures 1QFU.PDB and 1EO8.PDB). According to the authors, antibodies HC45 and BH151 are less prone to antigenic drift in HA and capable of binding more different HA mutants/subtypes. Therefore, in one embodiment the HBD in a proteinaceous substance of the invention comprises the VL and VH chains of a mouse monoclonal antibody (mAb), preferably mAb HC45 or BH151. However, it will be understood that, in principle, any VL-VH domain can be used which can bind to a broad range of HA subtype via an epitope that is not subject to antigenic drift, and thus will not act as neutralizing antibody.

HC45 is particularly useful for practicing the present invention because it shows the highest binding affinities for a range of HA subtype. The VL and VH chains of this antibody can be combined into a single-chain antibody (scHC45) by introducing a linker between the C-terminus of the VL and the N-terminus of the VH chain.

Preferred linkers to connect VL and VH chains in a proteinaceous substance of the invention are those being essentially resistant to proteolytic degradation and providing enough flexibility for the VL and VH chains to interact. Especially suitable is the "218" linker (Whitlow, M et al. 1993, Protein Eng. 6: 989-995). For example, the amino acid sequence of construct scHC45 (252 amino acids) is as follows (HC45 VL [bold] and VH [underlined] domain with the "218" linker-sequence [italics] in between): 
DVVMTQTPLSLPVSLGDQASISCRSSQTLVHSNGNTYLHWYLQKPGQSPKLL
IYKVSNRFSGVPDRFSGSGSGTDFTLKISRVEAEDLGVYFCSQNTHVPYTFGG
 
GTKLEIKGSTSGSGKPGSGEGSTKGQVQLQQPGAELVRPGASVKLSCKASGYTLT
TYWMNWFKQRPDQGLEWIGRIDPYDSETHYNQKFKDKAILTVDRSSSTAYMQLSS
LTSEDSAVYYCTRFLQITTIIYGMDYWGQGTSVTVSS
 In a further embodiment, the HA-binding domain is a so-called single-domain antibody (dAb) or camelised antibody. The variable part of heavy chains of normal antibodies (VH) can be used for binding if the contact residues with the light chain are to be replaced by more hydrophilic side chains to mimic camelid antibody heavy chains naturally devoid of light chain partner and to prevent aggregation. This is called camelising and results in a camelised heavy chain (cVH). Therefore, the following amino acid changes need to be placed into the heavy chain: L11S, G44E, L45R, W47G and V37F/Y (Davies, J. and Riechmann, L. 1996. Protein Eng. 9: 531-537; Muyldermans, S. 2001. J. Biotechnol. 74: 277-302).
The inventors herein provide a camelised variant of the HC45 mouse domain, resulting in a cVH domain (cVH45) with amino acid sequence (129 aa): 
QVQLQQPGAESVRPGASVKLSCKASGYTLTTYWMNWFKQRPDQEREGIGRIDPY
DSETHYNQKFKDKAILTVDRSSSTAYMQLSSLTSEDSAVYYCTRFLQITTIIYGMDY
WGQGTSVTVSSAKTTPPS
 In bold are the amino acid substitutions that render the VH domain more soluble. The underlined part is the VH domain. In italics is the spacer that connects the VH domain with the constant domain in the antibody. It is used here to connect the VH domain to the rest of the proteinaceous substance.
WO2007/011216 discloses polypeptides comprising a peptidoglycan binding domain fused to one or more antigen binding domains, such as an antibody capable of binding an antigen of interest. Exemplary antigens include polypeptides, carbohydrates, lipids, polynucleotides and pathogenic antigens, such as inactivated viral particles and purified antigenic determinants. However, the simultaneous presence of, on the one hand, a conserved influenza antigen, and, on the other hand, a binding domain for an influenza antigen that is subject to a high mutation rate, within a single polypeptide is not disclosed or suggested in the art.
As said, a proteinaceous substance according to the invention is characterized by the presence of, inter alia, a conserved influenza protein, for example a conserved nucleoprotein (NP) or a conserved matrix protein (M). Conserved influenza protein sequences can be found using the methods described by Heiny AT et al. 2007. PLoS ONE 2(11): e1190. For example, the proteinaceous substance comprises one or more (tandem) copies of influenza conserved matrix M1(58-66) peptide (Plotnicky, H et al. 2003. Virology 309:320-329).

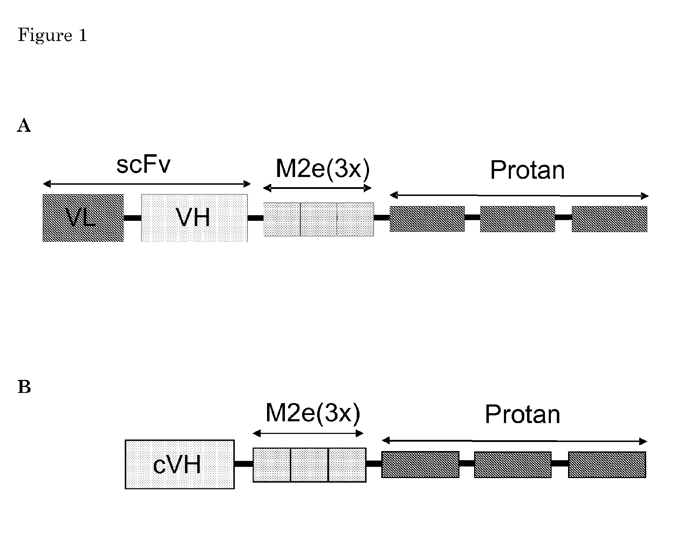

In one aspect, it comprises the extracellular part of a conserved influenza membrane protein. Preferably, it comprises the extracellular part of influenza membrane protein M2 (M2e), preferably M2e of influenza virus type A. Very suitable is the use of the conserved 23 amino acid extracellular peptide domain of the M2-protein (M2e) of influenza A. A broad-spectrum vaccine candidate based on said conserved 23 amino acid extracellular peptide domain of M2e has been developed (De Filette, M et al. 2005. Virology 337:149-161). Since the M2e peptide is poorly immunogenic, it is preferred to include more than one copy of the peptide in a proteinaceous substance of the invention, for example in a dimer or trimer configuration (see Figure 1). Such strategies were used previously when an M2e trimer was fused e.g. to the Hepatitis B core antigen (HBcAg). M2e-HBcAg vaccines were immunogenic and protective in mouse models, but still required additional adjuvant (De Filette, M et al. 2006. Vaccine 24:544-551).
One of the other characteristic elements of a proteinaceous substance provided herein is the immunostimulating carrier binding domain (IBD) which can link the (antigen-loaded) HA binding domain and the conserved influenza antigen (e.g. M2e trimer) to an immunostimulating carrier. In one embodiment, the IBD is a peptidoglycan binding domain capable of binding to an immunostimulating carrier comprising an accessible peptidoglycan surface. For example, the proteinaceous substance comprises an amino acid sequence selected from the group consisting of a LysM domain, an amino acid sequence retrieved from a homology search in an amino acid sequence database with a LysM domain in the C-terminus of *Lactococcus lactis* cell wall hydrolase AcmA (said domain herein also referred to as AcmA LysM domain) and a sequence showing at least 70% sequence identity to an AcmA LysM domain,
In one embodiment, the IBD comprises an amino acid sequence capable of binding to peptidoglycan, which sequence is a LysM domain. Preferably, a polypeptide comprises at least two, more preferably at least three LysM domains. The LysM (lysin motif) domain is about 45 residues long. It is found in a variety of enzymes involved in bacterial cell wall degradation (Joris et al. 1992. FEMS Microbiol. Lett. 70:257-264). The LysM domain is assumed to have a general peptidoglycan binding function. The structure of this domain is known ('The structure of a LysM domain from *E. coli* membrane-bound lytic murein transglycosylase D (MltD)'. Bateman A, Bycroft M. 2000. J. Mol. Biol. 299:1113-11192). The presence of the LysM domains is not limited to bacterial proteins. They are also present in a number of eukaryotic proteins, whereas they are lacking in archaeal proteins. A cell wall binding function has been postulated for a number of proteins containing LysM domains. Partially purified muramidase-2 of *Enterococcus hirae*, a protein similar to AcmA and containing six LysM domains, binds to peptidoglycan fragments of the same strain. The p60 protein of *Listeria monocytogenes* contains two LysM domains and was shown to be associated with the cell surface. The muropeptidases LytE and LytF of *Bacillus subtilis* have three and five repeats, respectively, in their N-termini and are both cell wall-bound.

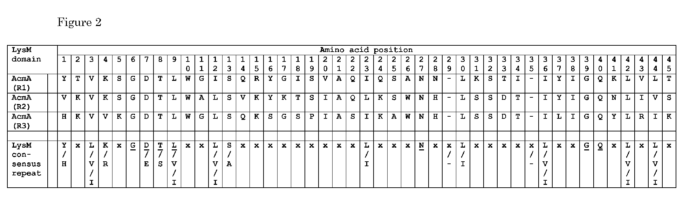

A skilled person will be able to identify a LysM domain amino acid sequence by conducting a homology-based search in publicly available protein sequence databases using methods known in the art. A variety of known algorithms are disclosed publicly and a variety of publicly and commercially available software can be used. Examples include, but are not limited to MacPattern (EMBL), BLASTP (NCBI), BLASTX (NCBI) and FASTA (University of Virginia). In one embodiment, PFAM accession number PF01476 for the LysM domain (see http://www.sanger.ac.uk/cgi-bin/Pfam/getacc?PF01476) is used to search for an amino acid sequence which fulfils the criteria of a LysM domain. The PFAM website provides two profile hidden Markov models (profile HMMs) which can be used to do sensitive database searching using statistical descriptions of a sequence family's consensus. HMMER is a freely distributable implementation of profile HMM software for protein sequence analysis.
The C-terminal region of the major autolysin AcmA of *L. lactis* contains three homologous LysM domains, which are separated by nonhomologous sequences. For the amino acid sequences of the three AcmA LysM domains see for example Figure 2 (essentially taken from WO99/25836) wherein the three LysM domains are indicated by R1, R2 and R3). The C-terminal region of AcmA was shown to mediate peptidoglycan binding of the autolysin (Buist, G. et al. 1995. J. Bacteriol. 177:1554-1563).
In one embodiment, the IBD of a proteinaceous substance of the invention comprises at least one LysM domain as present in AcmA. Variations within the exact amino acid sequence of an AcmA LysM domain are also comprised, under the provision that the peptidoglycan binding functionality is maintained. Thus, amino acid substitutions, deletions and/or insertions may be performed without losing the peptidoglycan binding capacity. Some parts of the AcmA LysM domains are less suitably varied, for instance the conserved GDTL, N and GQ motifs found in most LysM domains (Figure 2). Others may however be altered without affecting the efficacy of the IBD to bind the immunostimulating carrier. For example, amino acid residues at positions which are of very different nature (polar, apolar, hydrophilic, hydrophobic) amongst the three LysM domains of AcmA can be modified. Preferably, the IBD comprises a sequence that is at least 70%, preferably 80%, more preferably 90%, like 92%, 95%, 97% or 99%, identical to one of the three LysM domains of *L. lactis* AcmA. The IBD of a proteinaceous substance for use in the present invention may contain one or more of such (homologous) AcmA LysM domains, either distinct or the same. Typically, the LysM domains are located adjacent to each other, possibly separated by one or more amino acid residues. The LysM domains can be separated by a short distance, for example 1-15 amino acids apart, or by a medium distance of 15-100 amino acids, or by a large distance, like 150 or even 200 amino acids apart.
In a certain aspect, the invention concerns a IBD, comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to an AcmA LysM domain.

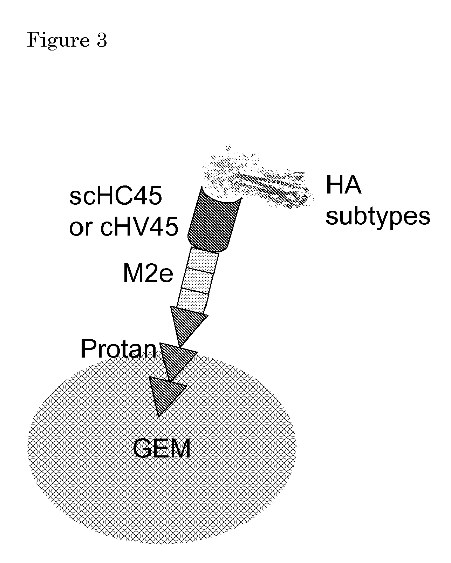

The 'percentage of amino acid sequence identity' for a polypeptide, such as 70, 80, 90, 95, 98, 99 or 100 percent sequence identity may be determined by comparing two optimally aligned sequences over a comparison window, wherein the portion of the polypeptide sequence in the comparison window may include additions or deletions (i.e. gaps) as compared to the reference sequence (which does not comprise additions or deletions) for optimal alignment of the two amino acid sequences. The percentage is calculated by: (a) determining the number of positions at which the identical amino acid occurs in both sequences to yield the number of matched positions; (b) dividing the number of matched positions by the total number of positions in the window of comparison; and (c) multiplying the result by 100 to yield the percentage of sequence identity. Optimal alignment of sequences for comparison may be conducted by computerized implementations of known algorithms, or by inspection. Readily available sequence comparison and multiple sequence alignment algorithms are, respectively, the Basic Local Alignment Search Tool (BLAST) (Altschul, S.F. et al. 1990. J. Mol. Biol. 215:403; Altschul, S.F. et al. 1997. Nucleic Acid Res. 25:3389-3402) and ClustalW programs both available on the internet.
In another embodiment, an IBD comprises a LysM domain which is present in an amino acid sequence retrieved from a homology search in an amino acid sequence database with an AcmA LysM domain, wherein the LysM domain is capable of attaching the substance to the cell wall of a Gram-positive microorganism. Preferably, the amino acid sequence retrieved is an amino acid sequence originating from a Gram-positive bacterium. It is for instance an amino acid sequence of a bacterial cell wall hydrolase. Preferably, the retrieved amino acid sequence shows at least 70%, more preferably 80%, most preferably at least 90% sequence identity with an AcmA LysM domain. Examples of sequences that may be retrieved can be found in Figure 11 of patent application WO99/25836.
Other aspects of the invention relate to a nucleic acid sequence encoding a proteinaceous substance according to the invention, and to an (expression) vector comprising said sequence. This allows inter alia for the production of a proteinaceous substance in a host cell, which host cell is accordingly also encompassed herein. Suitable host cells include *Lactococcus* ssp., *Lactobacillus* ssp., *Bacillus* ssp., *Streptococcus carnosus, Escherichia coli, Caulobacter crescentus, Saccharomyces* ssp., *Pichia* ssp., *Kluyveromyces* ssp., *Schizophyllum* ssp., *Trichoderma* ssp., *Streptomyces* ssp.,and mammalian cells such as Vero cells, CHO cells, MDCK cells, PerC6. In one embodiment, *Lactococcus* ssp. host cell comprising a nucleic acid sequence or a vector according to the invention is provided. In another embodiment, the host cell is a *Pichia* ssp.. If necessary, the nucleic acid sequence is designed and optimized for the codon usage of the host in order to increase the production levels of the encoded protein.
Still a further aspect relates to a proteinaceous substance according to the invention which substance is provided (via the at least one HBD) with an antigenic subtype of HA. This so-called 'preloaded polypeptide' is suitably attached to an immunostimulating carrier.
Accordingly, the invention also provides an immunostimulating carrier provided with a proteinaceous substance according to the invention, and methods for providing the same. The substance may be preloaded with HA antigen prior to being attached to the immunostimulating carrier. Alternatively, it is of course also possible to first attach a multifunctional protein linker to the carrier and subsequently load it with HA antigen. Thus, the invention furthermore provides an antigen-loaded immunostimulating carrier, wherein HA antigen is bound to the HBD of the proteinaceous substance that is attached to the carrier (see Figure 3 for an exemplary embodiment).

A method for providing an HA loaded immunostimulating carrier may comprise the steps of providing an immunogestimulating carrier; providing a proteinaceous substance comprising (i) at least one hemagglutinin (HA) binding domain (HBD) capable of binding to multiple antigenic subtypes of HA, (ii) a conserved influenza protein, or an antigenic fragment thereof, and (iii) an immunostimulating carrier binding domain (IBD) allowing attachment of said polypeptide to said carrier; and contacting said immunostimulating carrier and said proteinaceous substance; optionally comprising contacting said proteinaceous substance with an antigenic HA subtype. The step of providing an immunostimulating carrier preferably comprises preparing non-viable spherical peptidoglycan particles from a Gram-positive bacterium. Providing said multifunctional proteinaceous substance may comprise selecting an HA-binding domain from an antibody library, preferably using phage display technology. The proteinaceous substance is advantageously produced in a host cell by recombinant expression of a nucleic acid construct encoding said substance. Depending on the host cell of choice, the method may comprise harvesting the proteinaceous substance from the culture medium of the host cell or from a host cell extract or lysate.

As used herein, the term 'immunostimulating carrier' refers to a moiety which, upon administration to a subject, has the capacity to enhance or modify the immune-stimulating properties of an antigen attached to it. An immunostimulating carrier thus has adjuvant properties. Furthermore, it comprises peptidoglycans to allow attachment of one or more proteinaceous substance(s) via a peptidoglycan binding IBD. Non-recombinant immunostimulating carriers are preferred. In a preferred embodiment, the immunostimulating carrier complex is a non-viable spherical peptidoglycan particle obtained from a Gram-positive bacterium (GEM particle, or 'ghost'). Methods for the preparation of GEM particles have been described before, for instance in patent applications WO 02/101026 and WO 2004/102199. The process preserves most of the bacteria's native spherical structure. Briefly, the method comprises treating Gram-positive bacteria with a solution capable of removing a cell-wall component, such as a protein, lipoteichoic acid or carbohydrate, from the cell-wall material. The resulting GEM particles may be subsequently stored until it is contacted with a desired (antigen loaded) multifunctional protein anchor linker. GEM particles bind substantially higher amounts of an IBD than untreated Gram-positive bacteria. Therefore, a high loading capacity can be achieved for antigens on GEM particles (WO 02/101026). GEM particles are also better able to bind to and/or are more easily taken up by specific cells or tissues than mechanically disrupted cell-wall material. The ability of GEM particles to target macrophages or dendritic cells enhances their functional efficacy. The non-recombinant, non-living immunostimulating carrier complex of the present invention is therefore well suited as a vaccine delivery vehicle. See also WO 02/101026 and WO2004/102199.

The GEM particles can in principle be prepared from any Gram-positive bacterium. The cell walls of Gram-positive bacteria include complex networks of peptidoglycan layers, proteins, lipoteichoic acids and other modified carbohydrates. Chemical treatment of the bacterial cell-wall material may be used to remove cell-wall components such as proteins and lipoteichoic acids to result in GEM particles with improved binding characteristics. Preferably, an antigen binding immunostimulating carrier complex of the invention comprises GEM particles obtained using an acid solution (see e.g. WO 02/101026).
In a preferred embodiment, the immunostimulating carrier is prepared from a non-pathogenic bacterium, preferably a food-grade bacterium or a bacterium with the G.R.A.S. (generally-recognized-as-safe) status. In one embodiment, the cell-wall material is derived from a *Lactococcus*, a *Lactobacillus*, a *Bacillus* or a *Mycobacterium ssp*. Use of a Gram-positive, food-grade bacterium, such as *Lactococcus lactis,* offers significant advantages over use of other bacteria, such as *Salmonella* or *Mycobacterium*, as a vaccine delivery vehicle. *L. lactis* does not replicate in or invade human tissues and reportedly possesses low intrinsic immunity (Norton P.M. et al. 1996. FEMS Immunol. Med. Microbiol. 14:167-177). *L. lactis* expressing tetanus toxin fragment C has been shown to induce antibodies after mucosal delivery that protect mice against a lethal challenge with tetanus toxin even if the carrier bacteria were killed prior to administration (Norton P.M. et al. 1997. Vaccine 15:616-619). In contrast to the non-recombinant GEM particles in an immunostimulating carrier disclosed herein, these bacteria still contain recombinant DNA that will be spread into the environment, especially when used in wide-scale oral-immunization programmes. This uncontrollable shedding of recombinant DNA into the environment may have the risk of uptake of genes by other bacteria or other (micro) organisms.
As indicated above, one specific aim of the invention is to provide a versatile and flexible system for the annual reformulation of influenza vaccines. By virtue of binding multiple proteinaceous substances to an immunostimulating carrier, each substance capable of binding a broad range of HA subtypes it is possible to formulate an adjuvanted (e.g. GEM-based) influenza vaccine in which the HA antigen of all three strains is attached to a carrier without modification of the HA antigen production process. Therefore, in one embodiment the invention provides an antigen-loaded immunostimulating carrier, comprising at least two, preferably at least three, distinct antigenic subtypes of HA. Preferably, it comprises HA of two different influenza type A strains and HA of one influenza type B strain.
Another aspect relates to a pharmaceutical composition comprising an antigen-loaded immunostimulating carrier as disclosed herein, and a pharmaceutically acceptable carrier. It is for instance a vaccine composition formulated for mucosal administration, preferably for intranasal or oral administration. In one embodiment, a vaccine is a trivalent influenza vaccine, preferably a trivalent influenza vaccine for mucosal administration. Most currently used influenza vaccines are administered via the parenteral route. However, intranasal vaccines are attractive since influenza infections start from mucosal sites in the upper respiratory tract. The benefit of such a route of delivery is that mucosal vaccination resembles more closely natural infection. The respiratory tract is not only the site of infection for influenza but also the site of defence against influenza virus infection. Immune responses induced following intranasal immunization may prevent the penetration and replication of the virus in the mucosa at a very early stage of host invasion.

The invention provides in particular an adjuvanted vaccine. An appropriate adjuvant co-administered with the vaccine is usually required to achieve strong mucosal protection against invading pathogens. These adjuvants presumably exert their immunostimulatory effect by interacting with and activating a variety of immune cells, like macrophages, B-cells, and T-cells. GEM particles constitute such an adjuvant/carrier system. The carrier/adjuvant system of the present invention for instance consists of GEM particles. These particles act on the innate immune system through the toll-like receptors, most specifically but not limited to the TLR2 receptor. Recognition by TLRs, like TLR2 results in the activation and maturation of murine and human neonatal and adult antigen presenting cells such as dendritic cells. In preclinical animal studies, GEM particles stimulate systemic and local immune responses to proteinaceous antigens even to some extend when mixed with polypeptide antigen (Audouy S.A. et al. 2006. Vaccine 24:5434). The GEM particles provide adjuvant activity in mucosal and in parenteral vaccines. Mucosal administration of vaccines results often in local antibody responses even at sites distant from the administration site, e.g. GEM-based vaccines administered intranasally, elicit secretory IgA antibodies in the vagina. In the case of influenza the local response in the respiratory tract is pivotal to prevent entry of the virus into the body.

The invention furthermore relates to a method of prophylactic treatment of influenza in a subject in need thereof, comprising administering to the subject an effective dosage amount of a pharmaceutical composition of the invention. Preferably, said administering comprises the mucosal application of a vaccine. The subject is for instance a human subject at high or increased risk of developing severe, life threatening disease if infected with influenza. People at risk include children, the elderly, health care workers, immunocompromised individuals, and patients with cardiac disease. Also, the (prophylactic) treatment of avian influenza ('bird flu') in birds and mammals, especially humans, is encompassed.

### LEGENDS TO THE FIGURES

Figure 1: Schematic representation of exemplary proteinaceous substances comprising a trimer of the conserved influenza protein fragment M2e, an HA-binding domain consisting of either a single chain variable fragment (scFv; panel A) or a camelised heavy chain (cVH; panel B), and a peptidoglycan binding domain (Protan) capable of binding to an immunostimulating carrier.
Figure 2: Amino acid sequences of AcmA repeats 1-3 (R1, R2 and R2) and of the LysM consensus sequence. X denotes any naturally occurring amino acid. A dash (-) indicates an amino acid deletion at the indicated position. The amino acids underlined in the consensus sequence indicate the highly conserved motifs referred to in the text on page 9.
Figure 3: Schematic representation of an immunostimulating carrier comprising peptidoglycans (GEM) to which an HA-loaded proteinaceous substance is attached. In the embodiment shown, the substance is attached to the carrier via its peptidoglycan binding domain (Protan), a conserved influenza membrane protein (three copies of M2e) and an HA-binding domain.

## Claims

1. A proteinaceous substance comprising
(i) at least one hemagglutinin (HA) binding domain (HBD) capable of non-covalently binding to multiple antigenic subtypes of HA, (ii) a conserved influenza protein, or an antigenic fragment thereof, and (iii) an immunostimulating carrier binding domain (IBD).

2. Proteinaceous substance according to claim 1, wherein said HBD is capable of binding to multiple HA subtypes originating from influenza virus type A and/or type B.

3. Proteinaceous substance according to claim 1 or 2, wherein said HBD is an antibody or functional fragment thereof.

4. Proteinaceous substance according to claim 3, wherein said HBD comprises a camelised VH chain (cVH).

5. Proteinaceous substance according to claim 3, wherein said HBD comprises the VH and VL chains of a mouse monoclonal antibody (mAb), preferably mAb HC45 or BH151.

6. Proteinaceous substance according to claim 5, wherein said VH and VL chains are linked by a proteolysis-resistant flexible linker, preferably wherein said linker comprises the amino acid sequence Gly-Ser-Thr-Ser-Gly-Ser-Gly-Lys-Pro-Gly-Ser-Gly-Glu-Gly-Ser-Thr-Lys-Gly.

7. Proteinaceous substance according to any one of the preceding claims, wherein the conserved influenza protein is conserved nucleoprotein, a conserved matrix protein or a conserved membrane protein.

8. Proteinaceous substance according to claim 7, wherein the conserved influenza protein is a conserved extracellular part of an influenza membrane protein, preferably M2 of influenza virus type A.

9. Proteinaceous substance according to any one of claims 1 to 8, comprising at least two, preferably at least three repeats of said conserved influenza protein or antigenic fragment thereof.

10. Proteinaceous substance according to any one of the preceding claims, wherein said IBD is a peptidoglycan binding domain comprising an amino acid sequence selected from the group consisting of a LysM domain, an amino acid sequence retrieved from a homology search in an amino acid sequence database with a LysM domain in the C-terminus of *Lactococcus lactis* cell wall hydrolase AcmA (said domain herein also referred to as AcmA LysM domain) and a sequence showing at least 70% sequence identity to an AcmA LysM domain.

11. Proteinaceous substance according to claim 10, wherein said IBD comprises the C-terminal peptidoglycan binding domain of the *Lactococcus lactis* cell wall hydrolase AcmA.

12. A nucleic acid sequence encoding a proteinaceous substance according to any one of claims 1 to 11.

13. A vector comprising a nucleic acid sequence according to claim 12.

14. A host cell comprising a nucleic acid sequence according to claim 12 or a vector according to claim 13.

15. Proteinaceous substance according to any one of claims 1 to 11, wherein said at least one HBD is bound to an antigenic subtype of HA.

16. An immunostimulating carrier provided with a proteinaceous substance according to any one of claims 1 to 11.

17. Immunostimulating carrier according to claim 16, being a non-viable spherical peptidoglycan particle obtained from a Gram-positive bacterium (GEM particle).

18. Immunostimulating carrier according to claim 17, wherein said bacterium is a non-pathogenic bacterium, preferably a food-grade bacterium, more preferably wherein said bacterium is selected from the group consisting of a *Lactococcus*, a *Lactobacillus*, a *Bacillus* and a *Mycobacterium ssp.*

19. Antigen-loaded immunostimulating carrier, comprising an immunostimulating carrier according to any of claims 16-18, wherein at least one antigenic subtype of HA bound to the HBD of the proteinaceous substance.

20. Antigen-loaded immunostimulating carrier according to claim 19, comprising at least two, preferably at least three, distinct antigenic subtypes of HA are bound to the HBD.

21. Antigen-loaded immunostimulating carrier according to claim 19 or 20, comprising HA of two different influenza type A strains and HA of one influenza type B strain.

22. A pharmaceutical composition comprising an antigen-loaded immunostimulating carrier according to any one of claims 19-21 and a pharmaceutically acceptable carrier.

23. Pharmaceutical composition according to claim 22, being a vaccine composition formulated for mucosal administration, more preferably intranasal administration.

24. Pharmaceutical composition according to claim 22 or 23, being a trivalent influenza vaccine for use in humans.
